# EUROPEAN PATENT APPLICATION

(11) **EP 1 287 852 A1**
(43) Date of publication of application: **05.03.2003**
(21) Application number: 01936818.2
(22) Date of filing: 30.05.2001
(51) Int. Cl.: A61N 2/12

(54) **MAGNETICALLY THERAPEUTIC INSTRUMENT**

(30) Priority: 05.06.2000 JP 2000167333
(71) Applicant: Takenaka, Hiroshi, Ogaki-shi, Gifu 503-0982 (JP)
(72) Inventor: MIYAZAKI, Naokazu, Saga-shi, Saga 849-0924 (JP)
(74) Representative: Müller-Boré & Partner Patentanwälte
(86) International application number: JP0104545
(87) International publication number: WO01093954

(57) **Abstract**

A magnetically therapeutic instrument carries out highly efficient treatment with a simple structure. The magnetically therapeutic instrument generates a magnetic field for treatment in front of, for example, a south pole end 20s of a treating bar magnet 20 by rotating the treating bar magnet 20, and has a magnetism reflecting member 30 that covers at least a front part of the treating bar magnet 20, preferably approximately the whole of the treating bar magnet 20. Lines of magnetic force that enter or leave the front part of the treating bar magnet 20 are concentrated in front of the front opening of the magnetism reflecting member 30 due to the presence of the magnetism reflecting member 30, and the magnetic field density is increased.

## Description

### TECHNICAL FIELD

The present invention relates to a magnetically therapeutic instrument which generates a magnetic field to restore eyesight, treat decayed teeth, or carry out other treatment by means of rotation of a treating magnet.

### BACKGROUND ART

In recent years, there has been progress in the development of magnetically therapeutic instruments that carry out various types of treatment using a magnetic field generated by a treating magnet. For example, in Japanese Patent Application Laid-open No.H11-285539, a magnetically therapeutic instrument is disclosed in which a bar magnet and a motor are housed in a prescribed case, and the motor rotates the bar magnet at high speed about the axis joining the north and south poles of the bar magnet, thus causing fluctuations in the magnetic field generated by the bar magnet, and hence generating a magnetic field suitable for treatment in front of the south pole of the bar magnet.

To obtain a good treatment effect with such a magnetically therapeutic instrument, it is necessary to secure at least a certain magnetic flux density on the front side of the magnetically therapeutic instrument, i.e., on the side of the magnetically therapeutic instrument that is made to face the subject of the treatment. As means for increasing the magnetic flux density, there can be seen (1) using of a stronger treating magnet, and (2) increasing the speed of rotation of the treating magnet. However, in both cases either the treating magnet or the driving motor becomes larger, which is inconvenient in that it is an obstacle to making the magnetically therapeutic instrument as a whole small and inexpensive.

In view of such problems, it is an object of the present invention to provide a magnetically therapeutic instrument that makes possible highly efficient treatment with a simple structure.

### DISCLOSURE OF THE INVENTION

To solve the above problem, the following construction is adopted in the present invention.

The present invention is directed to a magnetically therapeutic instrument rotates a treating magnet having a north pole and a south pole about an axis joining the north pole and the south pole and thus generates a magnetic field for treatment in front of one of the north pole and the south pole. The magnetically therapeutic instrument includes a magnetism reflecting member that has a cylindrical shape with openings in the direction of the axis of rotation of the treating magnet and that covers at least a front part of the treating magnet from outside in the direction of the radius of rotation of the treating magnet, and is constructed such that lines of magnetic force that enter or leave the front part of the treating magnet are concentrated in front of the front opening of the magnetism reflecting member by the magnetism reflecting member.

According to this construction, at least the front part of the treating magnet is covered by the magnetism reflecting member from the outside, and hence lines of magnetic force that enter the front part (in the case that the front part is the south pole) or leave the front part (in the case that the front part is the north pole) must basically pass through the front opening of the magnetism reflecting member. The lines of magnetic force are thus concentrated in front of the front opening, and hence the magnetic flux density is increased here. That is, polarization between the north and south poles of the treating magnet is promoted, and as a result the treatment effect due to the magnetism in front of the treating magnet is increased.

It should be noted that the magnetism reflecting member does not necessarily need cover the front part of the treating magnet entirely, but rather, for example, the front part of the treating magnet may be partially exposed outside the magnetism reflecting member. The important thing is that the magnetism reflecting member should be disposed such that, due to the presence of the magnetism reflecting member, polarization between the north and south poles of the treating magnet is promoted and lines of magnetic force are concentrated at the front.

In the present invention, it is preferable for a partitioning member comprising a diamagnetic material to be fixed at a boundary part between the north pole and the south pole of the treating magnet, and for the magnetism reflecting member to be provided in at least a region in front of the partitioning member. As a result, lines of magnetic force are prevented by the partitioning member from passing from the north pole through the inside of the magnetism reflecting member and entering the south pole, and hence the above-mentioned polarization is further promoted, and the magnetic flux density in front of the magnetism reflecting member is further increased.

The shape of the magnetism reflecting member can be set to be any of various shapes, and may be for example a straight cylinder; however, if the magnetism reflecting member has a front widening part for which the diameter of a part covering the front part of the treating magnet widens out towards the front, then the lines of magnetic force reflected by the magnetism reflecting member can be concentrated in front of the front opening of the magnetism reflecting member yet more positively.

It is more preferable for the magnetism reflecting member to have a shape that covers approximately the whole of the treating magnet. By covering not only the front part but also the rear part of the treating magnet with the magnetism reflecting member in this way, the polarization between the north and south poles becomes yet more marked, and hence the efficiency is further increased.

In this case, it is yet more preferable for the magnetism reflecting member to have a front widening part for which the diameter of a part covering the front part of the treating magnet widens out towards the front, and a rear widening part for which the diameter of a part covering the rear part of the treating magnet widens out towards the rear.

Moreover, if the magnetism reflecting member has on the inside thereof a bearing part that rotatably supports the treating bar magnet, then the magnetism reflecting member can be utilized effectively to support the treating magnet so that the treating magnet can be rotated in a stable state.

As a specific example of means for rotating the treating magnet, it is suitable if a motor that rotates the treating magnet is provided to the rear of the treating magnet, and the magnetically therapeutic instrument has a casing that houses the motor and the magnetism reflecting member.

In the case that the magnetically therapeutic instrument has such a case, by adopting a construction in which the magnetism. reflecting member is fixedly provided inside the case in a state in which an outside part of the magnetism reflecting member is in contact with an inside surface of the casing, the magnetism reflecting member can be installed stably with a simple structure.

The magnetism reflecting member may be anything that has a property of reflecting magnetism and thus concentrating magnetic flux in front of the front opening; specifically, suitable examples include a magnetism reflecting member in which at least a surface thereof is composed of a diamagnetic material, a magnetism reflecting member in which a surface thereof facing the south pole part of the treating magnet is magnetized as a south pole and a surface thereof facing the north pole part of the treating magnet is magnetized as a north pole, or a magnetism reflecting member having fixedly attached to an inside surface thereof a plurality of magnetism reflecting magnets arranged in the peripheral direction so as to surround the treating magnet. The magnetism reflecting magnets are disposed such that a south pole part of each of the magnetism reflecting magnets faces the south pole part of the treating magnet and a north pole part of each of the magnetism reflecting magnets faces the north pole part of the treating magnet.

### BRIEF DESCRIPTION OF THE DRAWINGS

Fig. 1 is a sectional elevation view of an entirety of a magnetically therapeutic instrument according to an embodiment of the present invention;
Fig. 2 is a sectional elevation view showing main parts of the magnetically therapeutic instrument;
Fig. 3 is an explanatory view showing the magnetic field generated by the treating bar magnet provided in the magnetically therapeutic instrument;

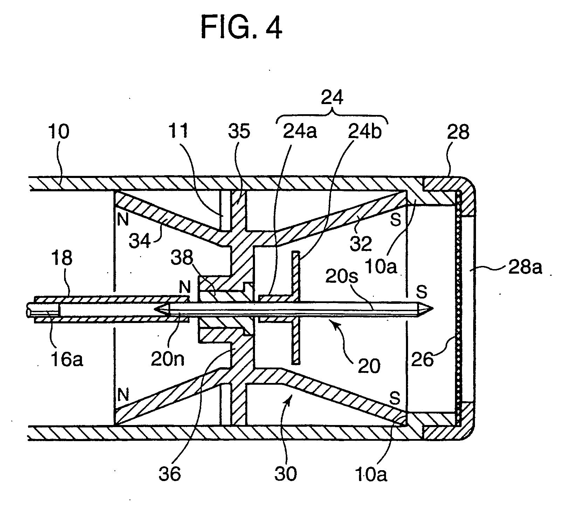

Fig. 4 is a sectional elevation view showing a variation of the magnetism reflecting member provided in the magnetically therapeutic instrument;
Fig. 5A is a sectional elevation view showing a variation of the magnetism reflecting member provided in the magnetically therapeutic instrument;
Fig. 5B is a sectional view along line A-A shown in Fig. 5A; and
Fig. 6 is a sectional elevation view showing a variation of the magnetism reflecting member provided in the magnetically therapeutic instrument.

### BEST MODE FOR CARRYING OUT THE INVENTION

A description will now be given of preferable embodiments of the present invention with reference to the drawings.

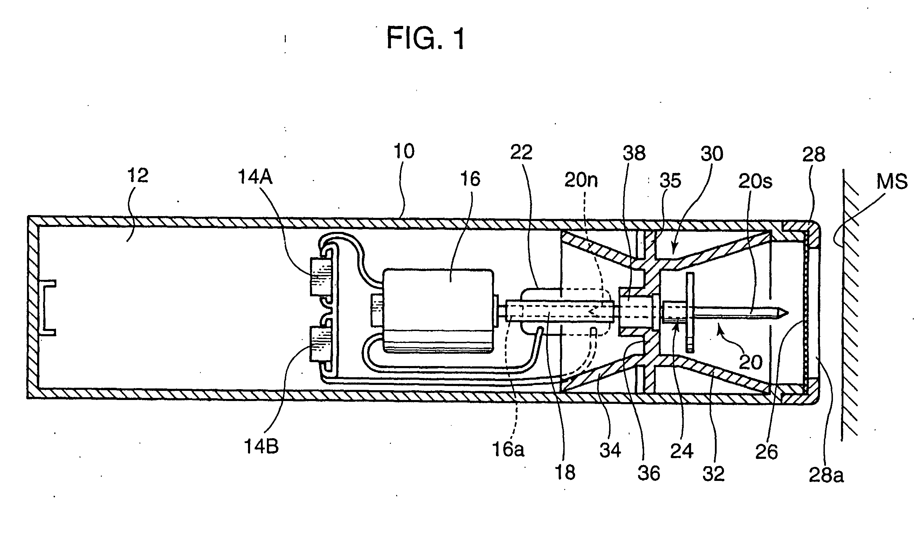

The magnetically therapeutic instrument shown in Fig. 1 has a casing 10, and a battery-housing space 12 is formed in a rear part of the casing 10. In front of the battery-housing space 12 are housed a pair of battery terminals 14A and 14B, a motor 16, a relaying shaft 18, and a treating bar magnet 20. A power switch 22 is provided on an outer surface of the casing.

The power source terminals 14A and 14B are provided in positions so as to come into contact with the electrodes of a battery installed in the battery-housing space 12. The power source terminal 14A is connected to one of the electrodes of the motor 16, and the other electrode of the motor 16 is connected to one of the contacts of the power switch 22. The other contact of the power switch 22 is connected to the power source terminal 14B. By operating the power switch 22, the power supply to the motor 16 from the battery can thus be switched on and off.

The motor 16 is disposed such that an output shaft 16a thereof points forwards. The relaying shaft 18 and the treating bar magnet 20 are disposed in positions coaxial with the output shaft 16a, and the treating bar magnet 20 is coupled to the output shaft 16a via the relaying shaft 18. Specifically, the relaying shaft 18 has a cylindrical shape with a small bore, and the output shaft 16a of the motor 16 is pushed into one of the open ends of the relaying shaft 18, and a north pole end 20n of the treating bar magnet 20 is pushed into the other open end of the relaying shaft 18, so that a south pole end 20s of the treating bar magnet 20 points forwards. By operating the motor 16, the relaying shaft 18 and the treating bar magnet 20, which are integrated with the output shaft 16a of the motor 16, are thus rotated about the axis joining the north and south poles of the treating bar magnet 20.

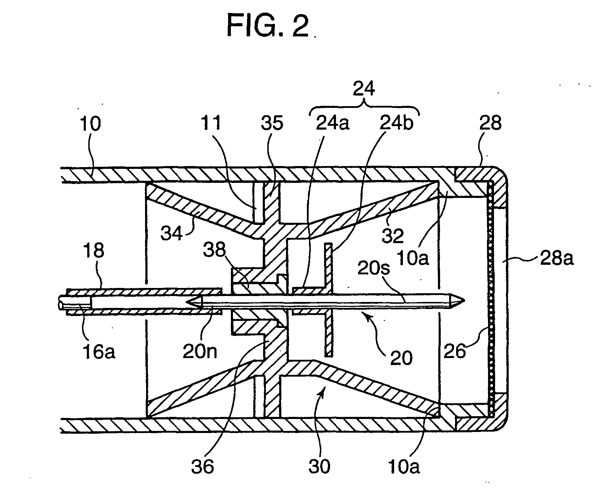

A partitioning member 24 comprising a diamagnetic material such as copper, silver or bismuth is provided at a boundary part between the north and south poles of the treating bar magnet 20, and as shown in Fig. 2 this partitioning member 24 has integrated together a cylindrical main body 24a having an inside diameter approximately the same as the outside diameter of the treating bar magnet 20 and a flange 24b that projects out from the outer peripheral surface of the cylindrical main body 24a in the radial direction, the cylindrical main body 24a is fixedly provided to the outer peripheral surface of the treating bar magnet 20 by means such as pushing in.

Moreover, a characteristic feature of the magnetically therapeutic instrument is that a magnetism reflecting member 30 is provided around the treating bar magnet 20.

Similarly to the partitioning member 24, the magnetism reflecting member 30 comprises a diamagnetic material, and as shown in Fig. 2 the magnetism reflecting member 30 has a shape so as to cover the treating bar magnet 20 from outside in the direction of the radius of rotation of the treating bar magnet 20 (i.e., a cylindrical shape with openings at the front and rear). A front widening part 32 whose diameter widens out towards the front is formed in the part of the magnetism reflecting member 30 that covers the front part of the treating bar magnet 20 (the south pole part in the drawings), and a rear widening part 34 whose diameter widens out towards the rear is formed in the part of the magnetism reflecting member 30 that covers the rear part of the treating bar magnet 20 (the north pole part in the drawings). In the example in the drawings, the south pole end 20s of the treating bar magnet 20 projects out slightly beyond the opening of the front widening part 32 (the front opening of the magnetism reflecting member 30).

A flange 35 that projects out from the outer peripheral surface of the magnetism reflecting member 30 is formed at the minimum diameter part of the magnetism reflecting member 30, i.e., the narrow part of the magnetism reflecting member 30 between the front widening part 32 and the rear widening part 34. Ribs 11 that project inwards are provided in suitable places on the inner surface of the casing 10, and a stepped part 10a where the casing 10 is narrower than elsewhere is formed at the front end of the casing 10. The front end of the magnetism reflecting member 30 projects out from the rear side against the stepped part 10a, and the flange 35 projects out from the front side against the ribs 11, and hence the magnetism reflecting member 30 is fixedly provided between the ribs 11 and the stepped part 10a in a state in contact with the inner peripheral surface of the casing 10.

Moreover, an approximately donut-shaped inner wall 36 having a boss part in the center thereof is formed on the inside of the above-mentioned minimum diameter part, and a bush 38 is fixedly provided to the inside of the inner wall 36. When the north pole part of the treating bar magnet 20 is inserted through the bush 38, the treating bar magnet 20 is supported by the bush 38 in such a way as to be rotatable. That is, a bearing part that rotatably supports the treating bar magnet 20 on the side of the magnetism reflecting member 30 is formed by the inner wall 36 and the bush 38.

Moreover, a mesh 26 that covers an opening at the front end of the casing 10 is provided at the front end of the casing 10. and a mesh restrainer 28 is installed on the casing 10 from above the mesh 26, thus holding the mesh 26 in place.

Next, a description will be given of the operation of the magnetically therapeutic instrument.

By switching the power switch 22 on with a battery installed in the battery-housing space 12, the motor 16 is operated, and hence the treating bar magnet 20 is rotated. As a result, fluctuations occur in the magnetic field around the treating bar magnet 20, and hence a magnetic field suitable for treatment is generated in front of the south pole end 20s of the treating bar magnet 20. In this state, by making the desired treatment surface MS come close to the front end of the magnetically therapeutic instrument as shown in Fig. 1, the treatment can thus be carried out.

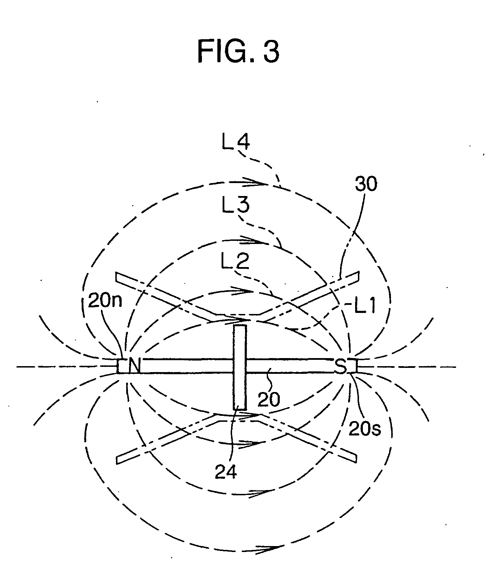

Here, in the case of a conventional magnetically therapeutic instrument, i.e., a magnetically therapeutic instrument without the magnetism reflecting member 30, of the lines of magnetic force L1 to L4 shown in Fig. 3 as representative examples, the only line of magnetic force that actually contributes to the treatment, i.e., the only line of magnetic force that passes through the space in front of the south pole end 20s, is L4, with the remaining lines of magnetic force L1 to L3 not passing through the space in front of the south pole end 20s and thus not contributing to the treatment of the treatment surface 40, and hence the efficiency is poor.

In contrast, if the magnetism reflecting member 30 is disposed around the treating bar magnet 20 as shown by the two-dot-and-dashed line in Fig. 3, then the lines of magnetic force L1 to L3 in Fig. 3 are reflected by the magnetism reflecting member 30, and hence can no longer follow the paths shown in Fig. 3, but rather can only enter the south pole by passing through the front opening of the magnetism reflecting member 30 (the opening on the right side in Fig. 3). All of the lines of magnetic force L1 to L4 are thus naturally concentrated together at the front opening of the magnetism reflecting member 30 (i.e., polarization of the north pole and the south pole is promoted), and the magnetic flux density in front of the front opening is increased. As a result, it becomes possible to increase the magnetic treatment effect without making the treating bar magnet 20 stronger or increasing the speed of rotation of the treating bar magnet 20.

In particular, as shown in the drawings, the magnetism reflecting member 30 is made to have a front widening part 32 that covers the front part of the treating bar magnet 20 and has a diameter that widens out towards the front. Consequently, due to the slope of the magnetism reflecting surface on the inside of the magnetism reflecting member 30, the lines of magnetic force can be concentrated to the front side yet more positively, resulting in yet more effective treatment.

Moreover, in the present embodiment, a bearing part comprising the inner wall 36 and the bush 38 is provided on the inside of the magnetism reflecting member 30, and hence by supporting the treating bar magnet 20 using this bearing part, the treating bar magnet 20 can be rotated in a more stable state.

In particular, by providing the bearing part 38 at the minimum diameter part of the magnetism reflecting member 30 as shown in the drawings, there is an advantage that the area of the inner wall 36 can be kept low.

Note that the magnetism reflecting member 30 may be anything that has a property of reflecting magnetism and thus concentrating the magnetic flux in front of the front opening; for example, it is possible to compose only the inner surface of the magnetism reflecting member 30 from a diamagnetic material, and use another metal or a non-metallic material such as a resin for the outside of the magnetism reflecting member 30.

Moreover, in place of the diamagnetic material, the magnetism reflecting member 30 may be a metallic material such as iron that has been magnetized in'advance as in Fig. 3.

In the case of the magnetism reflecting member 30 shown in the drawing, the front widening part 32 facing the south pole end 20s of the treating bar magnet 20 is magnetized as a south pole, and the rear side facing the north pole end 20n of the treating bar magnet 20 is magnetized as a north pole. According to this construction as well, the polarization of the treating bar magnet 20 is promoted by the repulsion between the north and south poles of the treating bar magnet 20 and the north and south poles of the magnetism reflecting member 30, and the magnetic flux is concentrated in front of the front opening, and hence the treatment effect can be increased.

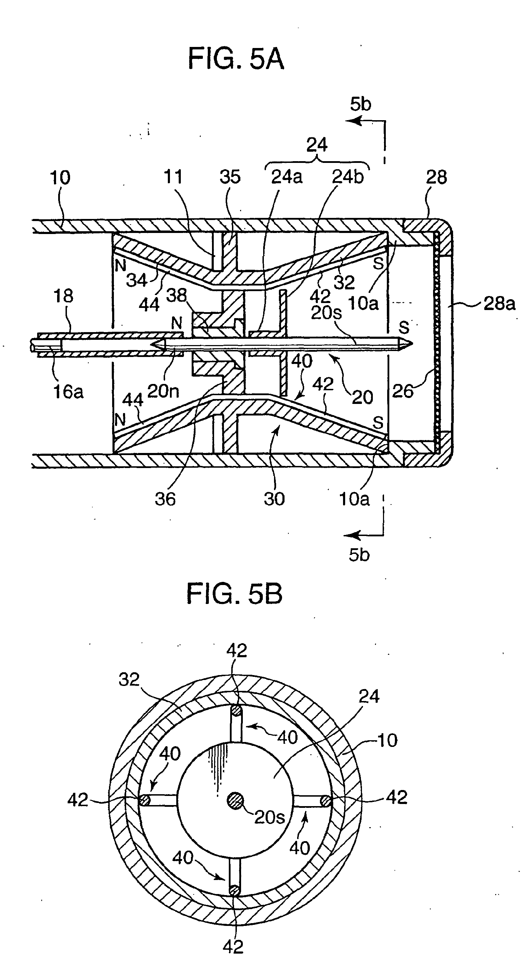

Alternatively, the magnetism reflecting member 30 may have a plurality of magnetism reflecting magnets 40 fixedly provided along the inner surface thereof, as shown in Fig. 5. The magnetism reflecting magnets 40 are arranged so as to surround the treating bar magnet 20, and such that a south pole part 42 of each of the magnetism reflecting magnets 40 faces the south pole end 20s, i.e., the front part, of the treating bar magnet 20 (in Fig. 5 the south pole part 42 is fixedly provided to the inner surface of the front widening part 32), and a north pole part 44 of each of the magnetism reflecting magnets 40 faces the north pole end 20n, i.e., the rear part, of the treating bar magnet 20 (in Fig. 5 the north pole part 44 is fixedly provided to the inner surface of the rear widening part 34).

According to this construction as well, the polarization of the treating bar magnet 20 is promoted by the repulsion between the north and south poles of the treating bar magnet 20 and the north and south poles of each of the magnetism reflecting magnets 40, and the magnetic flux density is concentrated in front of the front opening, and as a result a high treatment effect can be obtained. In this case, there are no particular limitations on the material of the main body part of the magnetism reflecting member 30, provided there is no adverse effect on the magnetic fields from the magnetism reflecting magnets 40; this material may be a non-metallic material such as a resin, or may be a diamagnetic material such as copper as mentioned previously.

Moreover, the present invention can adopt the following modifications.

In the embodiment shown in the drawings, a magnetically therapeutic instrument in which the south pole of the treating bar magnet 20 is made to point forwards was shown, but the north pole may be made to point forwards instead. The direction of rotation of the treating bar magnet 20 may also be-set as appropriate in accordance with the details of the treatment and so on.

In the present invention, the partitioning member 24 is not necessarily required to be provided. However, by providing the partitioning member 24, lines of magnetic force can be prevented from passing through the inside of the magnetism reflecting member 30, and hence the efficiency can be further increased.

The efficiency can be increased provided the magnetism reflecting member 30 covers at least the front part of the treating bar magnet 20, and hence, for example, the rear widening part 34 shown in Fig. 2 can be omitted. However, if the magnetism reflecting member 30 is made to have a shape so as to cover approximately the whole of the treating bar magnet 20 as shown in Fig. 2, then polarization between the north pole and the south pole of the treating bar magnet 20 can be promoted more.

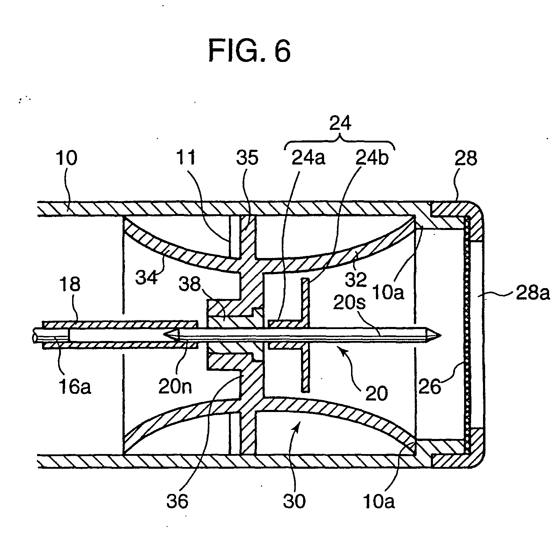

The shape of the magnetism reflecting member can be set to be any of various shapes, and may be for example a straight cylinder. However, by forming a front widening part 32 and a rear widening part 34 as shown in the drawings, better effects can be obtained as described above; even in this case, however, the widening shape is not limited to being conical as shown in Fig. 2, but may for example widen out in curved fashion as shown in Fig. 6.

### INDUSTRIAL APPLICABILITY

As described above, the present invention can provide a magnetically therapeutic instrument that carries out highly efficient treatment with a simple structure.

## Claims

1. A magnetically therapeutic instrument operable to rotate a treating magnet having a north pole and a south pole about an axis joining the north pole and the south pole to generate a magnetic field for treatment in front of one of the north pole and the south pole, the magnetically therapeutic instrument comprising a magnetism reflecting member including a cylindrical shape with openings in the direction of the axis of rotation of said treating magnet and covering at least a front part of said treating magnet from outside in the direction of the radius of rotation of said treating magnet for allowing lines of magnetic force entering or leaving the front part of said treating magnet to concentrate in front of the front opening of said magnetism reflecting member due to the presence of said magnetism reflecting member.

2. The magnetically therapeutic instrument according to claim 1, wherein a partitioning member made of a diamagnetic material is fixedly provided at a boundary part between the north pole and the south pole of said treating magnet, and said magnetism reflecting member is provided in at least a region in front of said partitioning member.

3. The magnetically therapeutic instrument according to claim 1, wherein said magnetism reflecting member has a front widening part for which the diameter of a part covering the front part of said treating magnet widens out towards the front.

4. The magnetically therapeutic instrument according to claim 1, wherein said magnetism reflecting member has a shape that covers approximately the whole of said treating magnet.

5. The magnetically therapeutic instrument according to claim 4, wherein said magnetism reflecting member has a front widening part for which the diameter of a part covering the front part of said treating magnet widens out towards the front, and said magnetism reflecting member has a rear widening part for which the diameter of a part covering a rear part of said treating magnet widens out towards the rear.

6. The magnetically therapeutic instrument according to claim 1, wherein said magnetism reflecting member has on the inside thereof a bearing part that rotatably supports said treating bar magnet.

7. The magnetically therapeutic instrument according to claim 1, wherein a motor for rotating said treating magnet is provided to the rear of said treating magnet, and the magnetically therapeutic instrument has a casing that houses said motor and said magnetism reflecting member.

8. The magnetically therapeutic instrument according to claim 7, wherein said magnetism reflecting member is fixedly provided inside said casing in a state in which an outside part of said magnetism reflecting member is in contact with an inside surface of said casing.

9. The magnetically therapeutic instrument according to any of claims 1 through 8, wherein at least a surface of said magnetism reflecting member is made of a diamagnetic material.

10. The magnetically therapeutic instrument according to claim 4, wherein said magnetism reflecting member has a surface thereof facing the south pole part of said treating magnet magnetized as a south pole, and a surface thereof facing the north pole part of said treating magnet magnetized as a north pole.

11. The magnetically therapeutic instrument according to claim 4, wherein said magnetism reflecting member has on an inside surface thereof a plurality of magnetism reflecting magnets arranged in a peripheral direction so as to surround said treating magnet, and said magnetism reflecting magnets are disposed such that a south pole part of each of said magnetism reflecting magnets faces the south pole part of said treating magnet, and a north pole part of each of said magnetism reflecting magnets faces the north pole part of said treating magnet.
